# EUROPÄISCHE PATENTSCHRIFT

(11) **EP 0 552 191 B1**
(45) Veröffentlichungstag und Bekanntmachung des Hinweises auf die Patenterteilung: **12.03.1997**
(21) Anmeldenummer: 91917243.7
(22) Anmeldetag: 30.09.1991
(51) Int. Cl.: C12P 13/04

(54) **MIKROORGANISMUS UND VERFAHREN ZUR GEWINNUNG VON ANTHRANILSÄURE**
MICRO-ORGANISM AND PROCESS FOR OBTAINING ANTHRANILIC ACID
MICRO-ORGANISME ET PROCEDE DE PRODUCTION D'ACIDE ANTHRANILIQUE

(30) Priorität: 08.10.1990 DE 4031854
(43) Veröffentlichungstag der Anmeldung: 28.07.1993
(73) Patentinhaber: BASF Aktiengesellschaft, 67063 Ludwigshafen (DE)
(72) Erfinder: COOPER, Bryan, D-6800 Mannhein 1 (DE); MEYER, Joachim, D-6717 Hessheim (DE); EULER, Klaus, D-6700 Ludwigshafen (DE)
(86) Internationale Anmeldenummer: EP9101863
(87) Internationale Veröffentlichungsnummer: WO9206207

(56) Entgegenhaltungen:
- CHEMICAL ABSTRACTS, vol. 113, no. 7, 13 August 1990, Columbus, Ohio US, abstract no. 57524E, page 563; H. KUMAGAI
- CHEMICAL ABSTRACTS, vol. 73, no. 23, 7 December 1970, Columbus, Ohio US, abstract no. 117487N, page 109; CH. PRASAD
- CHEMICAL ABSTRACTS, vol. 103, no. 1, 8 July 1985, Columbus, Ohio US, abstract no. 5024R, page 463; D.K.K. SHOWA

## Beschreibung

Die vorliegende Erfindung betrifft eine Mutante von Bacillus subtilis ATCC 6051 A mit der Hinterlegungsnummer DSM 6015 bei der Deutschen Sammlung von Mikroorganismen in Braunschweig sowie Mutanten von DSM 6015, welche wie DSM 6015 in einer L-Tryptophan enthaltenden Nährlösung Anthranilsäure erzeugen.

Es ist bekannt, daß Mikroorganismen zur fermentativen Gewinnung von Anthranilsäure (2-Aminobenzoesäure) eingesetzt werden. Dabei werden aber entweder nur geringe Konzentrationen an Anthranilsäure (JP-A 73/39692, JP-A 76/12992 und Agric. Biol. Chem., 49 (1985) 1151) oder zusätzlich noch Nebenprodukte wie Tryptophan, Indole oder Anthranilsäurederivate (JP-A 73/39692 und Cell. Mol. Biol., 26 (1980) 615) erzeugt, die die Aufarbeitung und Isolierung der Anthranilsäure erschweren.

Der Erfindung lag nun die Aufgabe zugrunde, einen Mikroorganismus bereitzustellen, der Anthranilsäure in höheren Konzentrationen und ohne störende Nebenprodukte produziert.

Demgemäß wurde die eingangs definierte Mutante DSM 6015 von Bacillus subtilis ATCC 6051 A gefunden.

Diese Mutante wurde am 19.06.90 bei der genannten Hinterlegungsstelle hinterlegt.

Außerdem wurde ein Verfahren zur fermentativen Herstellung von Anthranilsäure gefunden, welches dadurch gekennzeichnet ist, daß man die Mutante DSM 6015 des Stammes Bacillus substilis ATCC 6051 A in Gegenwart von L-Tryptophan kultiviert.

Die Mutante DSM 6015 läßt sich wie folgt taxonomisch beschreiben:
1. Gestalt der Zellen (Flüssigkultur in Nutrient Broth bei 30°C und 250 UpM beobachtet):
   Stäbchen mit Abmessungen 0,5 - 0,8 x 1 - 5 µm. Im Verlaufe des Wachstums treten unregelmäßige Formen auf, die wesentlich größer sein können.
2. Beweglichkeit: nicht beweglich.
3. Sporen: beobachtet.
4. Gram-Färbung: positiv in allen Wachstumsphasen.
5. Wachstum auf Minimalmedium + 1,1 Gew.-% Glucose: positiv in 2 bis 6 Tagen.
   Zusammensetzung des Minimalmediums:
   0,5 Gew.-% Ammoniumsulfat, 0,15 Gew.-% Kaliumdihydrogenphosphat, 0,36 Gew.-% Dikaliumhydrogenphosphat, 0,05 Gew.-% Magnesiumsulfat-7-hydrat, 0,005 Gew.-% Mangansulfat-1-hydrat, 0,2 Gew.-% Spurenelementlösung, 1,8 Gew.-% Agar, 0,005 Gew.-% L-Tryptophan, 0,00001 Gew.-% Biotin, 0,0001 Gew.-% Thiamin.
   Zusammensetzung der Spurenelementlösung ("SL"):
   200 mg/l Eisen(II)sulfat-monohydrat, 10 mg/l Zink(II)sulfat-4-hydrat, 3 mg/l Mangan(II)sulfat-1-hydrat, 30 mg/l Borsäure, 20 mg/l Cobalt-(II)chlorid-6-hydrat, 1 mg/l Kupfer(II)chlorid-2-hydrat, 2 mg/l Nickel(II)chlorid-6-hydrat, 3 mg/l Natriummolybdat-2-hydrat, 500 mg/l EDTA in destilliertem Wasser.
6. Vitaminbedarf: nicht vorhanden.
   Stimulierung des Wachstums auf Glucose-Minimalmedium durch Zugabe von 0,1 mg/l Biotin: stark ausgeprägt.
7. Aminosäurebedarf: L-Tryptophan
8. Koloniemorphologie: flache, glänzende, vorwiegend weiß-beige Kolonien.

Weitere charakteristische Eigenschaften entsprechen denen von Bacillus subtilis (s. Bergey's Manual of Systematic Bacteriology, Vol. 2, 1986, S. 1130).

Die erfindungsgemäße Mutante von Bacillus subtilis ATCC 6051 A erzeugt wie der Ausgangsstamm als weiteres, die Aufarbeitung nicht erschwerendes Produkt Acetoin (3-Hydroxy-2-butanon).

Man erhält die Mutante DSM 6015 durch mehrere hintereinander ausgeführte induzierte Mutationen mit mutagenen Substanzen wie N-Methyl-N'-nitro-N-nitroso-guanidin ("MNNG") oder durch UV-Bestrahlung und jeweils daran anschließende Selektionen. Dabei werden in einem ersten Schritt Mutanten selektiert, die trotz Zusatz des als Antimetabolit wirkenden 5-Fluor-DL-Tryptophans mehr Tryptophan erzeugen als sie für ihren eigenen Stoffwechsel benötigen. Anschließend werden in einem zweiten Schritt diese Tryptophan-produzierenden Mutanten weiter zur Resistenz gegen das als Antimetabolit wirkende Sulfaguanidin mutiert. In einer folgenden Mutation wurden Mutanten selektiert, die anstelle von Tryptophan Anthranilsäure ausscheiden und sich deshalb durch eine Fluoreszenz bei 366 nm detektieren lassen.

Man kann das erfindungsgemäße Verfahren nach der üblichen Technik diskontinuierlich oder kontinuierlich vornehmen, und zwar zweckmäßigerweise durch Inkubation bei pH 6 bis 8, vorzugsweise bei pH 6,5 bis 7,5, und einer Temperatur von 25 bis 40°C, vorzugsweise 28 bis 37°C, in einer flüssigen Nährlösung, die L-Tryptophan enthält. Gewöhnlich fermentiert man zur Anreicherung der Anthranilsäure auf eine Konzentration im Bereich von 2 bis 15, vorzugsweise 3 bis 10 g/l, 36 bis 72 Stunden lang.

Die Wahl des Nährmediums zur Züchtung des Mikroorganismus ist nicht kritisch. Es enthält neben L-Tryptophan die für diesen Mikroorganismus üblichen Kohlenstoff- und Stickstoffquellen sowie weitere für das Wachstum essentielle Stoffe.

Als Kohlenstoffquellen verwendet man beispielsweise Zucker, vorzugsweise Glucose, oder Zuckersäuren wie Gluconsäure. Als Stickstoffquelle kommen sowohl anorganische wie organische Verbindungen in Betracht, beispielsweise Ammoniumsalze, bevorzugt Ammoniumsulfat, Nitrate, Maisquellwasser, Hefeextrakt oder Peptone.

Üblicherweise enthält das Nährmedium noch Sulfate und/oder Phosphate der Elemente Magnesium, Mangan und Kalium, die Spurenelemente Eisen, Zink, Bor, Cobalt, Kupfer, Nickel und Molybdän, die beispielsweise in Form der oben genannten Spurenelementlösung eingesetzt werden können, sowie Vitamine wie Biotin und/oder Thiamin.

Bei der Fermentation setzt man L-Tryptophan in der Regel im Bereich von 5 bis 500 mg/l, bevorzugt von 10 bis 50 mg/l ein.

Das Mengenverhältnis der anderen genannten Nährstoffe hängt von der Art der Fermentation ab und wird im Einzelfall in an sich bekannter Weise festgelegt. Beispielsweise eignen sich zur Durchführung des erfindungsgemäßen Verfahrens Glukosekonzentrationen im Bereich von 50 bis 200 g/l, bevorzugt sind Konzentrationen im Bereich von 100 bis 150 g/l. Das als Stickstoffquelle bevorzugte Ammoniumsulfat setzt man im Bereich von 5 bis 50 g/l ein, besonders von 10 bis 30 g/l.

Den Zusatz des ebenfalls als Stickstoffquelle bevorzugten Hefeextraktes wählt man im Bereich von 0,5 bis 5 g/l.

Magnesiumsulfat-heptahydrat dient vorzugsweise als Magnesiumquelle und wird im allgemeinen im Bereich von 0,1 bis 5 g/l, insbesondere von 0,5 bis 2 g/l eingesetzt. Mangansulfat-Hydrat als bevorzugte Manganquelle, verwendet man im Bereich von 0,01 bis 1 g/l, vorzugsweise von 0,1 bis 0,5 g/l. Eine bevorzugte Kaliumquelle ist Kaliumdihydrogenphosphat, das man in Konzentrationen von 1 bis 10 g/l, bevorzugt von 2 bis 5 g/l, als Bestandteil des Nährmediums hinzufügt. Eine andere ist Dikaliumhydrogenphosphat, das man in Konzentrationen von 1 bis 15 g/l, besonders von 5 bis 10 g/l, dem Nährmedium zusetzt.

Die Spurenelemente verwendet man in der Regel in einem Konzentrationsbereich von jeweils 0,1 bis 1000 mg/l, besonders von 1 bis 300 mg/l als wäßrige Lösung, und zwar bevorzugt in der oben genannten Zusammensetzung.

Man kann die Nährlösung von vornherein mit der Gesamtmenge aller Nährstoffe versetzen, jedoch empfiehlt es sich, die Nährstoffe nach Maßgabe ihres Verbrauchs zu dosieren.

Man trennt Anthranilsäure aus der Fermentationsbrühe in der Regel dadurch ab, daß man die Fermentationsbrühe mit einer Mineralsäure wie Salzsäure oder Schwefelsäure auf einen pH-Wert von 1 bis 3, bevorzugt von 1,5 bis 2,5, ansäuert und dann mit einem organischen Lösungsmittel wie einem Ether, besonders Methyl-tert.butylether, oder einem Ester wie Essigsäureethylester nach bekannten Methoden, beispielsweise in einer Gegenstromextraktionsanlage, extrahiert. Anschließend ist es vorteilhaft, zunächst das Lösungsmittel destillativ zu entfernen. Dabei setzt man dem Gemisch zweckmäßigerweise vor der Destillation eine Base zu, vorzugsweise Natronlauge, um die Anthranilsäure in die Salzform zu überführen. Dadurch wird während der Destillation eine Kondensationsreaktion der Anthranilsäure mit dem vom Mikroorganismus ebenfalls erzeugten Acetoin verhindert. Nach der Destillation kann man die Anthranilsäure durch Ansäuern, vorzugsweise mit Salzsäure, wieder freisetzen. Die Anthranilsäure wird dann in der Regel durch Ausfällen und Filtration in einem Temperaturbereich von 5 bis 15°C isoliert, wobei das Ausfällen durch Zusätze von Lösungsmitteln wie Toluol, in denen die Anthranilsäure schwer oder nicht löslich ist, unterstützt werden kann. Das Nebenprodukt Acetoin kann man in der Regel durch Destillation aus dem verbliebenen Gemisch abtrennen.

Anthranilsäure ist ein vielseitig verwendetes Zwischenprodukt, beispielsweise bei der Herstellung von Pharmazeutika, Kosmetika und einer Reihe von Farbstoffen. Acetoin dient beispielsweise als Aromastoff zur Geschmacksverbesserung von Margarine.

### Beispiel 1

Züchtung und Isolierung der für die Erfindung geeigneten Mutante DSM 6015
a) Mutagenese des Ausgangsstammes
20 ml eines sterilen wäßrigen Nährmediums (im folgenden als Nährmedium I bezeichnet), welches folgende Zusammensetzung hatte:

| Nährmedium I: | |
|---|---|
| Glucose | 11 g/l |
| Ammoniumsulfat | 5 g/l |
| Magnesiumsulfat-Heptahydrat | 0,5 g/l |
| Mangansulfat-Hydrat | 0,05 g/l |
| Kaliumdihydrogenphosphat | 1,5 g/l |
| Dikaliumhydrogenphosphat | 3,6 g/l |
| Spurenelementlösung | 2 ml/l |
| Biotin | 0,1 mg/l |
| Thiamin | 1 mg/l |

wurden mittels einer öse mit dem Stamm Bacillus subtilis ATCC 6051 A beimpft und bei 30°C und 200 UpM auf einem Schüttelapparat inkubiert. Nach 16 h wurde dann 1 ml einer 4 gew.-%igen wäßrigen MNNG-Lösung hinzugegeben. Die Kultur wurde dann 10 min weitergeschüttelt. Danach wurde die gesamte Kultur abzentrifugiert, und der überstand wurde verworfen. Die abzentrifugierten Zellen wurden in 20 ml des auf 4°C gekühlten Nährmediums I dispergiert und erneut abzentrifugiert. Dieser Trennvorgang wurde wiederholt. Dann wurden die abzentrifugierten Zellen in 20 ml einer wäßrigen Lösung, die 10 Gew.-% Glycerin und 5 Gew.-% Lactose enthielt, aufgenommen. Diese Suspension enthielt 10⁷ lebensfähige Zellen pro 1 ml.
b) Selektion durch den Antimetaboliten 5-Fluor-DL-Tryptophan
Je 0,1 ml der unter a) erhaltenen Suspension wurden dann auf 100 Agarplatten, die das folgende Medium enthielten, verteilt: Nährmedium 1, 18 g/l Agar und 50 mg/l 5-Fluor-DL-Tryptophan. Die so präparierten Platten wurden dann bei 30°C 5 Tage bebrütet.
c) Isolierung und Vermehrung der selektierten Mutanten
Die jeweils 5 größten Kolonien, die auch Satellitenkolonien bildeten, wurden einzeln in 100 ml-Kolben, die jeweils 20 ml Nährmedium II, bestehend aus Nährmedium I und zusätzlich 11 g/l Glucose, 5 g/l Ammoniumsulfat und 0,2 g/l Soytone (standardisiertes Soya-Pepton der Fa. Difco), enthielten, überimpft und für zwei Tage bei 30°C mit 200 UpM geschüttelt. Nach dieser Zeit wurden mittels HPLC die Tryptophankonzentrationen in den jeweiligen Kolben bestimmt und diejenige Mutante Ml isoliert, die am meisten L-Tryptophan (0,2 g/l) unter diesen Bedingungen produzierte.
d) Steigerung der Tryptophan-Produktion
Die Mutante Ml wurde analog zu dem beschriebenen Verfahren einer Mutagenese unterworfen. Je 0,1 ml dieser danach erhaltenen Suspension wurden dann auf 50 Platten mit Nährmedium II, dem zusätzlich 18 g/l Agar und 200 mg/l Sulfaguanidin zugesetzt waren, ausplattiert. Die so präparierten Platten wurden dann bei 30°C 5 Tage inkubiert. Kolonien, die sich durch die Resistenz gegen Sulfaguanidin auszeichneten, wurden einzeln in 100 ml-Kolben, die jeweils 20 ml Nährmedium II enthielten, überimpft und für zwei Tage bei 30°C mit 200 UpM geschüttelt. Nach dieser Zeit wurden die Tryptophankonzentrationen wie oben bestimmt und diejenige Mutante M2 isoliert, die am meisten L-Tryptophan (0,5 g/l) produzierte.
e) Mutation und Selektion zur Erzeugung Anthranilsäure-produzierender Mutanten
Die Mutante M2 wurde ebenfalls einer Mutagenese nach dem unter a) beschriebenen Verfahren unterworfen. Die dabei erhaltene Suspension wurde wie unter b) angegeben 5 Tage bei 30°C bebrütet, mit dem Unterschied, daß das Nährmedium keinen Antimetaboliten enthielt. Die Selektion erfolgte durch Bestrahlung mit Licht der Wellenlänge 366 nm. Es wurden solche Kolonien isoliert, die dabei in blauer Farbe fluoreszierten. Sie wurden einzeln in 100 ml-Kolben, die jeweils 20 ml Nährmedium II und 10 mg/l L-Tryptophan enthielten, überimpft und für zwei Tage bei 30°C mit 200 UpM geschüttelt. Nach dieser Zeit wurden die Anthranilsäurekonzentrationen bestimmt und diejenige Mutante M3 isoliert, die am meisten Anthranilsäure (0,25 g/l) produzierte.
f) Steigerung der Anthranilsäure-Produktion
Analog zu dem unter e) beschriebenen Verfahren wurde die Mutante M3 mutiert und selektiert. Die dabei ausgewählten Kolonien wurden einzeln in 100-ml-Kolben, die jeweils 20 ml Nährmedium II und 500 mg/l L-Tryptophan enthielten, überimpft und für zwei Tage bei 30°C und 200 UpM geschüttelt. Danach wurden die Anthranilsäurekonzentrationen bestimmt und diejenige Mutante M4 isoliert, die am meisten Anthranilsäure (1 g/l) produzierte.
Die Mutante M4 wurde in einem 100-ml-Schüttelkolben mit 20 ml Nährmedium II und 10 mg L-Tryptophan bei 30°C und 200 UpM 2 Tage geschüttelt. Anschließend wurden je 0,1 ml der dabei erhaltenen Suspension auf 100 Agarplatten, die Nährmedium II, 500 mg/l L-Tryptophan und 18 g/l Agar enthielten, ausplattiert. Die so präparierten Platten wurden dann bei 30°C 3 Tage bebrütet. Die 200 größten Kolonien wurden jeweils in 100-ml-Kolben, die jeweils 20 ml Nährmedium II und 10 mg L-Tryptophan enthielten, bei 30°C und 200 UpM für 2 Tage geschüttelt. Hiervon wurde die Mutante M5, die 1,5 g/l Anthranilsäure und 10 g/l Acetoin produzierte, isoliert und unter der Bezeichnung DSM 6015 hinterlegt.

### Beispiel 2

### Herstellung von Anthranilsäure im Fermenter

Ein 14-Liter-Fermenter wurde mit 9,8 1 einer sterilen wäßrigen Nährlösung beschickt, welche folgende Bestandteile enthielt:

| | |
|---|---|
| Glucose | 1 200 g |
| Ammoniumsulfat | 200 g |
| Kaliumdihydrogenphosphat | 30 g |
| Dikaliumhydrogenphosphat | 72 g |
| Hefeextrakt | 10 g |
| Magnesiumsulfat-Heptahydrat | 10 g |
| Mangansulfat-Hydrat | 1 g |
| Spurenelementlösung | 40 ml |
| L-Tryptophan | 320 mg |

Eine Vorkultur wurde aus 200 ml der gleichen Nährlösung durch Beimpfen mit der Mutante DSM 6015 (vgl. Beispiel 1) hergestellt. Sie wurde 16 h bei 37°C und 200 UpM bebrütet. Der 14-1-Fermenter wurde mit dieser Vorkultur beimpft und bei 37°C und 750 UpM betrieben, wobei 1 Volumenteil Luft pro Volumenteil Reaktor und pro Minute durch die Lösung geleitet wurden. Dabei wurde der pH-Wert durch automatische Regelung mit einer 20 gew.-%igen NaOH-Lösung auf pH 7 eingestellt.

Nach 60 h wurde die Fermentation durch Ansäuern mit Schwefelsäure auf pH 2 abgebrochen. Die Konzentrationen der gebildeten Produkte wurden in üblicher Weise mittels HPLC bestimmt. Dabei betrug die Konzentration an Anthranilsäure 3,5 g/l, die des Acetoins 25 g/l in der Fermentationsbrühe.

Diese Lösung wurde mit 100 1 Methyl-tert.butylether bei Raumtemperatur extrahiert. Der organische Extrakt wurde dann mit 3,1 1 einer 1 N NaOH-Lösung versetzt, und anschließend wurde der Methyl-tert.butylether bei Normaldruck abdestilliert. Danach wurden der alkalischen Lösung 300 ml Toluol zugesetzt. Anschließend wurde die Lösung bei 10°C mit halbkonzentrierter Salzsäure auf pH 3,5 angesäuert und drei Stunden bei dieser Temperatur gerührt. Die ausgefallene Anthranilsäure wurde abfiltriert, mit 500 ml kaltem Toluol gewaschen und bei 1 mbar und 60°C vom Lösungsmittel befreit. Es fielen 29 g Anthranilsäure (charakterisiert mittels ¹H-NMR-Spektroskopie und HPLC) mit einem Schmelzpunkt von 133 bis 135°C (Lit.: 146.1°C, Chemikerkalender) und einer Reinheit von 94 % (HPLC) an.

Der Rückstand wurde einer Destillation unterworfen, wobei 115 g Acetoin mit einer Reinheit von 90 % (HPLC) erhalten wurden.

## Patentansprüche

1. Mutante von Bacillus subtilis ATCC 6051 A mit der Hinterlegungsnummer DSM 6015 bei der Deutschen Sammlung von Mikroorganismen in Braunschweig sowie Mutanten von DSM 6015, welche wie DSM 6015 in einer L-Tryptophan enthaltenden Nährlösung Anthranilsäure erzeugen.

2. Verfahren zur fermentativen Herstellung von Anthranilsäure, dadurch gekennzeichnet, daß man den Mikroorganismus DSM 6015 oder Mutanten davon gemäß Anspruch 1 in Gegenwart von L-Tryptophan kultiviert.

## Claims

1. A mutant of Bacillus subtilis ATCC 6051 A with the deposit number DSM 6015 at the Deutsche Sammlung von Mikroorganismen in Braunschweig, and mutants of DSM 6015 which, like DSM 6015, produce anthranilic acid in a nutrient solution containing L-tryptophan.

2. A process for preparing anthranilic acid by fermentation, which comprises cultivating the microorganism DSM 6015 or mutants thereof as claimed in claim 1 in the presence of L-tryptophan.

## Revendications

1. Mutante de Bacillus subtilis ATCC 6051 A avec le numéro de dépôt DSM 6015 auprès de la collection allemande de micro-organismes (Deutschen Sammlung von Mikroorganismen) à Braunschweig, ainsi que des mutantes de DSM 6015, qui, comme DSM 6015, produisent de l'acide anthranilique dans une solution nutritive contenant du L-tryptophane.

2. Procédé de préparation par fermentation de l'acide anthranilique, caractérisé en ce que l'on cultive le micro-organisme DSM 6015 ou des mutantes de celui-ci suivant la revendication 1, en présence de L-tryptophane.
